# EUROPEAN PATENT APPLICATION

(11) **EP 1 767 937 A1**
(43) Date of publication of application: **28.03.2007**
(21) Application number: 05291998.2
(22) Date of filing: 27.09.2005
(51) Int. Cl.: G01N 33/50

(54) **Method of diagnosis of tuberculosis related immune restoration syndrome (IRS)**

(71) Applicant: INSTITUT NATIONAL DE LA SANTE ET DE LA RECHERCHE MEDICALE (INSERM), 75013 Paris (FR)
(72) Inventor: Autran, Brigitte, 75017 Paris (FR); Bourgarit, Anne, 75011 Paris (FR); Carcelain, Guislaine, 92130 Issy Les Moulineaux (FR); Martinez, Valérie, 75015 Paris (FR); Gicquel, Brigitte, 75014 Paris (FR); Sereni, Daniel, 75005 Paris (FR)
(74) Representative: Warcoin, Jacques

(57) **Abstract**

The present invention relates to a method and kit of diagnosis of Immune Restoration Syndrome (IRS) in patients co-infected with tuberculosis (TB) and HIV comprising detecting an increase in Th1 response following exposure to mycobacterial extract of the tuberculin bacillus, referred as PPD, and no increase with ESAT-6, CFP-10 and/or 85B.

## Description

The present invention relates to a method and kit of diagnosis of Immune Restoration Syndrome (IRS) in patients co-infected with tuberculosis (TB) and HIV comprising detecting an increase in Th1 response following exposure to mycobacterial extract, referred as tuberculin or PPD (Purified Protein Derivative), but not to ESAT-6 or CFP-10, two antigens from mycobacterium tuberculosis.

### Background of the invention

First described in HIV-infected patients with *Mycobactrium avium complex* infection receiving zidovudine monotherapy [1], Immune restoration syndrome (IRS) in patients suffering from tuberculosis became extremely frequent (29-36%) [2] with the Highly Active Antiretroviral Therapy (HAART) era. IRS can also occur after initiation of TB therapy in HIV-negative patients, though at a lower frequency.

Classically, IRS occurs in patients co-infected with tuberculosis (TB) and HIV, naïve of HAART, with advanced HIV disease, when both therapies are initiated simultaneously or at a short interval. Usually, clinical symptoms include fever, lymphadenopathy, worsening of respiratory and other initial TB symptoms [2]. Definition IRS major criteria have been proposed [3] whatever the underlying opportunistic event including an atypical presentation of opportunistic infection (OI) or tumours in patients responding to HAART (decrease in plasma HIV RNA level more than 1log₁₀ copies/ml), without any alternative explanation (drug resistance, other OI) and with evidence for immune restoration, as minor criteria.

However, IRS raises three clinically relevant issues : first, diagnosis is difficult as IRS should be distinguished from treatment failure or resistance or with other opportunistic diseases; second, IRS treatment in some life-threatening cases is still empirically based on steroids, which are uneasy to introduce in such immunocompromised patients; third, determination of IRS risk factors could help clinicians to determine their therapeutic strategy, i.e. delaying or not HAART introduction [4].

Therefore, there is still as of today a strong need for a reliable test for diagnosis of IRS.

The pathophysiology of IRS is not yet totally elucidated. Acute proinflammatory cytokine (IL-6) production has been demonstrated during IRS [5, 6] but its source remains unknown. In addition, a strong and dominant hypothesis is that IRS would be associated to acute restoration of mycobacteria-specific T-lymphocyte response with either recirculation or proliferation of memory T-cells after HAART has reduced the plasma viral load (VL) [7, 8]. This hypothesis is only suggested but not yet demonstrated, except for the restoration of DTH skin test to PPD [9]. Similarly in HIV-negative TB-infected patients, it is hypothesized that IRS reflects exacerbation of TB-specific Th1 immunity.

In connection with the invention, we conducted a prospective multicenter study of patients co-infected with HIV and TB. We found that Mycobacteria-specific (PPD) Th1 IFN-γ-producing cells increased sharply during IRS, while the CMV-, HIV p24-specific responses tested as controls did not. In addition, only very few IRS+ patients had ESAT-6-specific responses albeit at low levels during IRS; and IRS- patients did not develop acute PPD-specific responses. These findings correlated with a peak of PPD-specific Th1 cytokines such as IFN-γ, IL-2, IL-12, IP10 and MIG but we did not detect Th2 cytokines; other inflammatory chemokines or cytokines were also detected such as TNF-α, IL-6, IL-1β, IL-10, RANTES and MCP-1.

Thus, we provide here a new diagnostic test of IRS consisting of detecting the acute exacerbation of Th1 responses against mycobacterial antigens in patients co-infected with HIV and TB as well as patients with TB alone.

### Description

In a first embodiment, the invention is aimed at a method of in vitro diagnosis of Immune Restoration Syndrome (IRS) in patients co-infected with mycobacterium tuberculosis (TB) as well as in patients infected with TB alone, comprising the step consisting of:
a) incubating peripheral blood mononuclear cells (PBMC) of said patients in a culture medium with mycobacterial antigens including mycobacterial extract referred as PPD (purified protein derivative), and at least one antigen from mycobacterium tuberculosis selected from ESAT-6, CFP-10 and 85B;
b) detecting the level of the Th1 response against said mycobacterial antigens;
wherein an acute increase of Th1 response against said PPD and no or minimal reactivity with ESAT-6, CFP-10 and/or 85B compared to non-stimulated cells or control cells stimulated with non-mycobacterial antigens is indicative of IRS.

By acute increase of Th1 response, it will be understood herein an increase typically comprised between 500% and 5000% above background or at least above 250% of Th1 response compared to control cells. By "no or minimal reactivity", it will be understood insignificant increase of the Th1 response compared to control cells, for example below 200% and typically below 100% or 50%.

PPD, 85B and CFP-10 are antigens isolated from mycobacterium tuberculosis well known in the art and can be obtained from several commercial sources including the PPD marketed by Statens Serum Institute, Copenhagen, Denmark. PPD is found in PPD vaccine which is a tuberculin vaccine containing a purified protein derivative of the tuberculin bacillus commonly employed in testing for the presence of antibodies against tubercle bacilli. For example, it can be obtained from the Tubersol® of Connaught Laboratories Limited prepared from a large Master Batch, Connaught Tuberculin (CT68). The ESAT-6 protein is a major T-cell antigen which has been purified from M. tuberculosis short-term culture filtrates as described in Harboe et al., 1996; Sorensen et al.,1995). As referred herein ESAT-6, CFP-10 and 85B can be obtained from cell lysate and purification or by recombinant technique. For example ESAT6 can be obtained as a recombinant protein from Statens Serum Institute.

Tuberculin or PPD (purified protein derivative) from mycobacterial extracts differs from ESAT-6 (early secreted antigenic target 6) CFP-10 (culture filtrate protein 10), and 85B which are encoded by genes located within the M. tuberculosis genome, because it is also containing other antigens that are shared with any BCG substrains, several nontuberculous mycobacterial species.

Step b) may comprise performing a quantification of antigen-specific T-cells by ELISPOT assay, such as a quantification of mycobacterial-specific Th1 cells producing IFN-γ. Here, a result above 500 SFC (spot-forming-cells)/10⁶ PBMC, for example a SFC above 500, 1000, or 2000, after subtraction of the mean background obtained with cells alone, is an indication of IRS; while simultaneously no or minimal SFC are detected against ESAT-6, 85B, CFP-10, such as SFC typically below 500/10⁶ PBMC or below 400 or 300.

The ELISPOT method can be replaced by an intra-cellular cytokine staining combined to flow cytometry or fluorescent microscopic examination and quantification.

Alternatively or concurrently step b) may comprise collecting culture supernatants of step a) and quantifying at least one Th1 specific cytokine or IL-2, IL-2R, IL-12p40, IL-15, IL-17, IFN-γ, TNF-α, GMCSF, MIP-1α, MIP-1β, MCP-1, MIG, RANTES, IP-10.

Such analysis can be performed using a multiplexed sandwich immunoassay or an individual cytokine-specific sandwich immuno-assay.

In one particular aspect of the invention, step b) comprises quantification of IFN-γ production by ELISA or RIA.

Preferably, peripheral blood mononuclear cells in step a) are incubated from about 6 to 72 hours. In addition, peripheral blood mononuclear cells in step a) are advantageously incubated with PPD at about 1 µg/ml and separately with ESAT-6 recombinant protein at about 1 µg/ml. PBMC may also be incubated with CFP-10 or 85B.

Control cells are selected from cells incubated with no antigen or non-mycobacterium antigen. For example, it is possible to use CMV extracts, HIV-1 p24, phytohaemagglutinin. Culture with medium alone serves as negative control to measure background

The method of the invention is performed in the course of Highly Active Antiretroviral Therapy (HAART) in HIV positive patients co-infected with tuberculosis. More specifically, it is performed in patients receiving simultaneous or sequential HAART and tuberculosis treatment.

HAART is well known in the art and includes several protocols of combined treatment with Nucleoside Analog Reverse Transcriptase Inhibitors (NARTIs) or (NRTIs), NonNucleoside Reverse Transcriptase Inhibitors (NNRTIs), and Protease inhibitors (PIs).

In a second embodiment, the invention is directed to a kit for performing the method as defined above.

Thus, the invention relates to a diagnostic kit of Immune Restoration Syndrome (IRS) in patients co-infected with tuberculosis (TB) and HIV, comprising mycobacterial antigens including mycobacterial extract referred as PPD (purified protein derivative), and at least one antigen from mycobacterium tuberculosis selected from ESAT-6, CFP-10 and 85B. For example, the kit comprises PPD and ESAT-6 or PPD, ESAT-6 and CFP-10 and/or 85B.

In addition, the kit may comprise at least one anti-cytokine/chemokine antibody specific to the Th1 immune response bound to a solid support, such as anti-IFN-γ antibodies. The solid supports of the kit can be an ELISPOT plate and/or an ELISA plate.

In a third embodiment, the invention is directed the use of the method and kit defined above for detecting Immune Restoration Syndrome (IRS) in patients co-infected with tuberculosis (TB) and HIV in course of HAART and tuberculosis treatment. It also concerns the use of the kit for detecting Immune Restoration Syndrome (IRS) in patients with TB alone.

### Figure legend

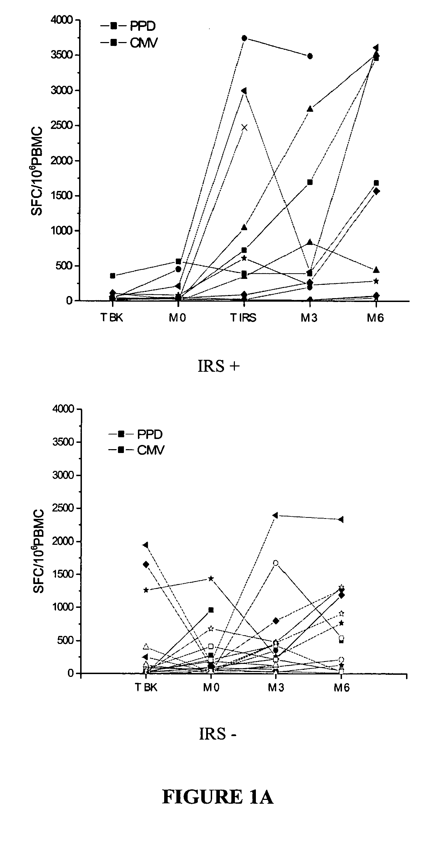

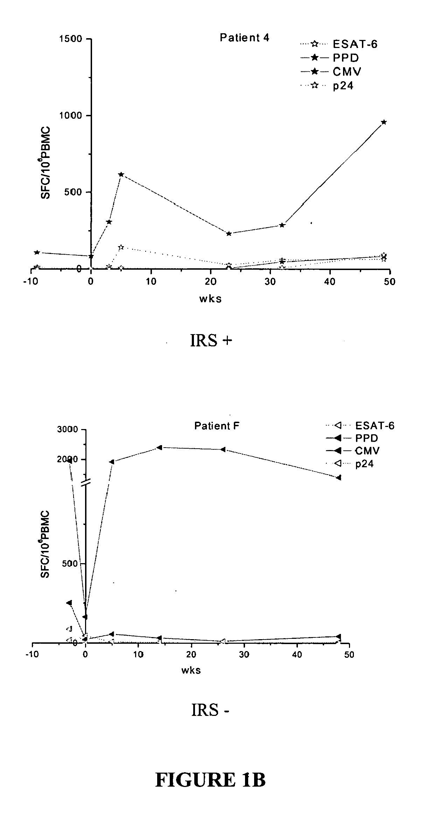

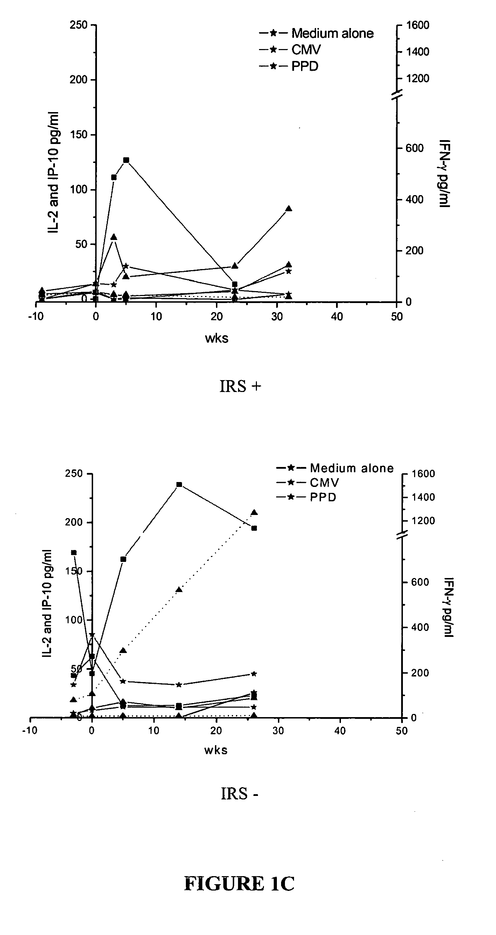

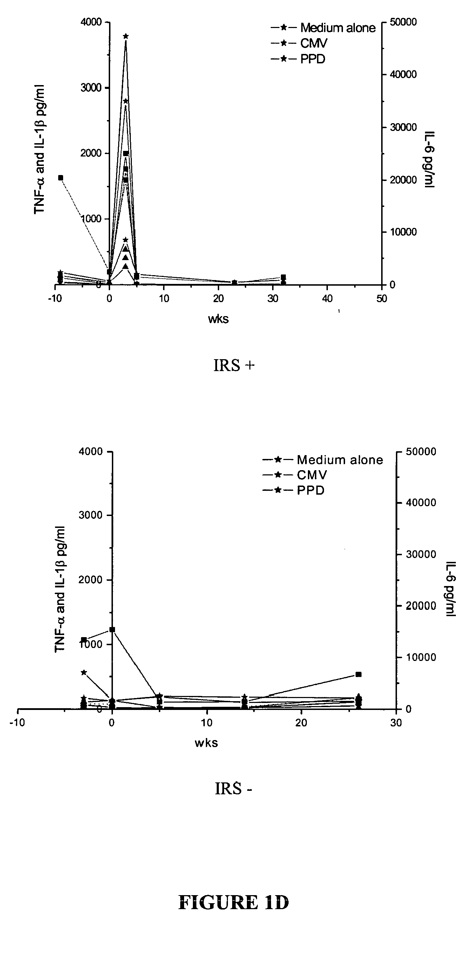

### Figure 1: PPD-specific reactivity between patients with and without IRS.

The left column represents data for IRS+ patients, and the right column data for IRS-patients.

A: Overall results of ELISpot IFN-γ assays expressed as Spot Forming Cells (SFC)/million PBMC performed in all IRS+ and IRS- patients after 40 hours PBMC stimulation with PPD (red) or CMV (cyan) **B, C, D**: Representative results from one IRS+ and one IRS- patient **B**: IFN-γ ELISpot after stimulation with PPD, ESAT6 (red) versus control antigens (CMV , HIV₁ p24) (cyan); **C, D**: Luminex-measured production of: **C**: IFN-γ (■), IL-2 (★), IP-10 (A) and **D**: TNF-α (★), IL-6 (■), IL-1β (▲), after in vitro PBMC stimulation with PPD (red), CMV (cyan) and medium alone in two representative IRS+ and IRS- patients.

### Example 1: IRS is induced by mycobacterial-specific Th1 cells in HIV-tuberculosis co-infected patients

### 1- Patients and methods.

### 1.1.1 Patients

Twenty-nine consecutive TB-HIV co-infected untreated patients were prospectively included when beginning anti-TB treatment. Inclusion criteria were : HIV-1 infection, no previous HAART, CD4 count below 200 cells/mm³, anti-TB therapy initiated within a maximum of one week before inclusion and indication to further HAART initiation. Inclusion was secondly confirmed when *M tuberculosis* infection was proved (positive culture or histological findings). Patients were evaluated when initiating antimycobacterial therapy (T_{BK}) and HAART (M₀) and at 1, 3, 6 and 12 months after HAART initiation (M_{1,3,6,12}). In addition, patients with IRS (IRS+) were evaluated at IRS time (T_{IRS}) and 20 days later. IRS was defined as follows [3] : recurrence of inflammatory reaction (fever, elevated CRP), enlargement of pre-existing lesions, or development of new lesions (lymph nodes, pleuritis) with no mycobacterium resistance, no positive culture specimen, no other diagnosis and with a response to HAART (HIV-RNA decrease >1 log copies/ml). Patients who did not experience IRS within three months after HAART initiation were defined as IRS-.
The study was accepted by the Saint-Louis Hospital Institutional-Ethical-Committee and all patients signed an informed consent.

### 1.1.2 Methods

### ELISPOT assay for quantification of mycobacterial-specific Th1 cells

Antigen-specific Th1 cells producing IFN-γ were prospectively quantified on fresh peripheral blood mononuclear cells (PBMC) by ELISpot as described [10], after a 40-hour stimulation with mycobacterial extracts (PPD, 1µg/ml; Statens Serum Institute, Copenhagen, Denmark) and ESAT-6 recombinant protein (1 µg/ml, kindly provided by Statens Serum Institute, Copenhagen, Denmark). Controls included CMV extracts (Behring), HIV-1 p24 (Protein-Sciences), phytohaemagglutinin (Murex) and medium alone. Spots were counted using an ELISpot reader (Zeiss) and data were expressed as spot-forming-cells (SFC)/10⁶ PBMCs. Results were considered as positive if above a 50 SFC/10⁶ PBMC after subtraction of the mean background obtained with cells alone.

### Cytokine/chemokine production quantification

Fresh PBMC were stimulated for cytokine/chemokine production with the same antigens as above. Culture supernatants were collected at day 2 of incubation and were cryopreserved. Quantification of 25 inflammatory and immunomodulatory cytokines/chemokines (IL-1β, IL-1RA, IL-2, IL-2R, IL-4, IL-5, IL-6, IL-7, IL-8, IL-10, IL-12p40, IL-13, IL-15, IL-17, IFN-γ, IFN-γ, TNF-α, GMCSF, MIP-1α, MIP-1β, MCP-1, MIG, RANTES, IP-10, Eotaxin) was performed for four patients (3 IRS + and 1 IRS-) using the multiplexed sandwich immunoassay (Human 25-plex®, Biosource) based on flowmetric Luminex® technology (Luminex 100^{™}, Clinisciences) as published [11]. The IFN-γ production was quantified on the same supernatants by a Biosource ELISA kit.

### Lymphocyte phenotyping

CD4 and CD8 T-lymphocyte phenotype was analysed in fresh whole blood by four-colour flow cytometry using standard methods [7] with the following antibodies: anti-HLA-DR-FITC (Immunotech), anti-CD25-PE (Becton Dickinson), anti-CD4-PerCPCy5 (Becton Dickinson) or anti-CD8-PerCP (Becton Dickinson) and anti-CD3-APC (Becton Dickinson). Stained cells were analysed on a FACScalibur (Becton Dickinson) as described.

### Statistic analysis

Comparisons between groups were made using χ² and Student t tests as appropriate.

### 1.2 Results

Twenty-nine consecutive TB-HIV co-infected untreated patients were prospectively included when they began anti-TB treatment. Ten were then excluded for the following reasons: four had unproven tuberculosis, five moved and one was rapidly transferred to the ICU. The other 19 were followed for at least three months after initiating HAART. Seven of them (39%) experienced IRS within a median of 23 (12-85) days after M₀. IRS clinical manifestations are listed in Table 1.

**TABLE 1:**

| Patient | Age yr | sex | TB localisation | CD4 /mm³ | VL log/ml | Time to HAAR T dy | HAART | ΔCD4 M₃-M₀ | IRS Clinical manifestations |
|---|---|---|---|---|---|---|---|---|---|
| 1 | 30 | F | pleuritis | 26 | 4.7 | 36 | 3TC TFV EFV | 86 | Peritonitis, tubal granuloma |
| 2 | 41 | M | liver, lymph nodes | 9 | 5.7 | 15 | AZT 3TC T20 | 125 | Fever, hepatitis |
| 3 | 43 | M | lung | 16 | 5.04 | 14 | AZT 3TC EFV | 15 | Pericarditis |
| 4 | 32 | F | lung, bone marrow, lymph nodes, liver | 15 | 6.5 | 63 | 3TC D4T NFV | 107 | Fever, abdominal lymph node swelling |
| 5 | 35 | M | lung, lymph nodes | 115 | 5.8 | 40 | AZT 3TC EFV | 94 | Fever, peritonitis, abdominal lymph node swelling |
| 6 | 32 | F | lung | 24 | 5.7 | 27 | FTC TFV EFV | 367 | Fever, alveolar pneumonit is, lymph node swelling |
| 7 | 49 | M | miliary, perihepatic lymph nodes | 6 | 5 | 10 | TFV FTC LPV/RTV | 14* | Fever, abdominal pain, lymph node swelling |
| A | 36 | M | lymph nodes | 15 | 4.96 | 39 | AZT 3TC ABC | 71 | None |
| B | 38 | M | lung, lymph nodes, spleen | 8 | 5.6 | 20 | AZT 3TC NFV | 53 | None |
| C | 38 | M | liver, lymph nodes, lung | 22 | 5.7 | 27 | AZT 3TC EFV | 20 | None |
| D | 37 | M | lung | 32 | 5.4 | 14 | AZT 3TC EFV | 49 | None |
| E | 30 | F | lung, liver, spleen | 43 | 4.8 | 61 | AZT 3TC ABC | -7 | None |
| F | 26 | F | abdominal lymph nodes, iliitis | 47 | 5.0 | 76 | AZT 3TC LPV/rtv | 47 | None |
| G | 34 | M | abdominal lymph nodes | 131 | 5.1 | 50 | TFV 3TC RTV ATZ | 80 | None |
| H | 31 | M | lung | 198 | 5.9 | 24 | AZT 3TC EFV | -88 | None |
| I | 61 | M | lung, lymph node | 166 | 5.5 | 111 | 3TC DDI EFV | 18 | None |
| J | 52 | M | lung, liver, spleen | 219 | 4.7 | 53 | 3TC ABC TFV DDI | 80 | None |
| K | 35 | M | lung | 267 | 5.2 | 23 | AZT 3TC ABC | 74 | None |
| L | 63 | M | lymph nodes, lung, genitourinary | 60 | 5.7 | 89 | AZT 3TC EFV | 354 | None |

Three patients received oral steroids therapy with rapid recovery; at time of IRS, HAART was transiently stopped for one of them. Patients with (IRS+) and without (IRS-) IRS did not differ for ethnicity, age, sex, dissemination of tuberculosis or time between T_{BK} and M₀ (27 vs. 45 days respectively, NS). Median CD4 counts at M₀ were lower, though not significantly, in IRS+ patients (median 32/mm³ for IRS+ vs. 60/mm³ for IRS-, p=0.053) and rose at M₃ by 107/mm³ in IRS+ versus 51/mm³ in IRS-patients (NS). Time to reach an undetectable viral load did not differ either.
The number of PPD-specific cells producing IFN-γ did not differ at baseline (M₀) between the two groups but their proportion increased sharply during IRS and reached a median of 2970 SFC/10⁶PBMC (range 616-3744) in the 7 IRS+ patients (fig 1A and B left panel). This explosive reactivity was restricted to this antigen and did not affect the CMV-specific responses in IRS+ patients. The IRS- patients did not develop acute PPD-specific responses (median 430 SFC/10⁶PBMC), except one patient who had an acute increase above 1500 SFC/10⁶PBMC at M₃ without clinical IRS; HAART at this time was no longer effective because of poor adherence. In addition, IRS- patients responded more to CMV than IRS+ patients (median 415 SFC/10⁶PBMC vs. 150 SFC/10⁶PBMC) (fig 1A and B).
When comparing the reactivity against the two mycobacteria antigens, we found that, contrasting with PPD reactivity, only 3 IRS+ patients had ESAT-6-specific positive responses during IRS with frequencies of 58, 142 and 318 SFC/10⁶ PBMC (figure 1B). Furthermore, only two patients without IRS, both with CD4>100/mm³, recognized the ESAT-6 antigen during follow-up (data not shown).
An extensive proteomic analysis was also performed for four patients (3 IRS+, 1 IRS-), to evaluate the in-vitro production of 25 inflammatory and immunomodulatory cytokines/chemokines by chemiluminescence in antigen-stimulated PBMC-supernatants. Two distinct patterns were observed during IRS. First, PPD-specific IFN-γ production peaked with that of other PPD-specific Th-1 related cytokines/chemokines (IL-2, IL-12, IP 10 and MIG) (Fig 1C) but no Th2 cytokine modulation occurred (IL-4, IL-5, IL-13, IL-15). Second, inflammatory cytokines/chemokines (TNF-α, IL-6, IL-1β, IL-10, RANTES and MCP-1) (Fig 1D) peaked, produced by both unstimulated and antigen-stimulated cells. In addition, PPD-specific cytokine/chemokine production far exceeded the CMV-specific response in IRS+ patients, while the CMV-reactivity predominated in the IRS- patient (Fig 1 D right).
Finally, whole-blood four-color flow cytometry analysis of CD3+ CD4+ and CD8+ T-cells showed that expression of activation markers (HLA-DR, CD25+) increased sharply during IRS, following exactly the same kinetics than the PPD-specific IFN-γ-producing responses (data not shown); while as expected, these markers decreased during HAART in patients without IRS.

### 1.3 Conclusion

The results of this prospective sequential study of HIV and TB co-infected patients initiating anti-TB and HAART therapies provide the first demonstration that IRS do correspond to an exacerbation of TB-specific Th1 responses with proinflammatory events during the immune reconstitution allowed by HAART.
It is well known that recovery of a PPD-specific response is a characteristic of immune reconstitution with HAART [12-15]. Our prospective study differs from others by clearly showing an exceptionally acute, intense and reproducible kinetic of the PPD-specific response concurrent with the acute inflammatory clinical symptoms described as IRS. This abrupt response was absent in only one IRS + patient who stopped HAART during IRS and received corticosteroids, while, one IRS- patient showed a similar peak though with no clinical diagnosis of IRS but with poor HAART observance and immuno-virological control. Such findings had not been demonstrated yet possibly due to the retrospective characteristics of previous studies.
In addition, we show here that the mycobacteria-specific response is limited to some persistent antigens contained in PPD. Indeed, the weak response to the mycobacterial secreted protein ESAT-6 suggests that ESAT-6 either does not persist after antimycobacterial therapy or does not stimulate the T-cells involved in IRS. This finding is critical for diagnosis because new tuberculosis-diagnostic tests based on ESAT-6 [16] would have missed this acute response.
The sharp amplification of IFN-gamma producing mycobacteria-specific T cells was associated with an acute burst of other Th1 and proinflammatory cytokines/chemokines and is in accordance with the results from Stone et al. [6] showing high levels of seric IL-6 produced in IRS+ patients. We show here that both phenomenon correlate with the abrupt onset of commonly reported clinical findings [2]: lymph node enlargement and functional granuloma formation [17]. We therefore propose a two step pathophysiology for IRS : the first event is an excessive restoration of PPD-specific Th1 response with no Th2 balance, responsible for enlargement of TB granuloma lesions, rapidly leading to an acute release of non-specific proinflammatory cytokines and chemokines inducing the systemic inflammatory syndrome. This latter findings support the use of systemic anti-inflammatory and/or immunosuppressive agents in severe IRS cases.
This mechanism can probably be extended to non-HIV patients experiencing equivalent paradoxical reactions during anti-TB therapy. In some non-HIV patients, IFN-γ secretion is missing during active TB infection, reappears during antimycobacterial treatment [18]. This desinhibition of IFN-γ production may be particularly strong concurrently to the restoration of immune response in profoundly immune-suppressed patients. No identified factors explain why this physiologic response to mycobacterial antigens becomes so intense and sometimes life-threatening in some patients, but genetic factors regulating Th1 functions may determine predisposition to IRS and its severity as already proposed by P. Price [19].
In conclusion, an acute exacerbation of Th1 responses against some mycobacterial antigens appears to cause IRS in patients co-infected with HIV and TB. This key event provides new evidence valuable for the diagnosis and treatment of IRS.

### REFERENCES

1. French MA, Mallal SA, Dawkins RL. Zidovudine-induced restoration of cell-mediated immunity to mycobacteria in immunodeficient HIV-infected patients. Aids 1992;6:1293-1297.
2. Lawn SD, Bekker LG, Miller RF. Immune reconstitution disease associated with mycobacterial infections in HIV-infected individuals receiving antiretrovirals. Lancet Infect Dis 2005;5:361-373.
3. French MA, Price P, Stone SF. Immune restoration disease after antiretroviral therapy. Aids 2004;18:1615-1627.
4. Dean GL, Edwards SG, Ives NJ, Matthews G, Fox EF, Navaratne L, et al. Treatment of tuberculosis in HIV-infected persons in the era of highly active antiretroviral therapy. Aids 2002;16:75-83.
5. Morlese JF, Orkin CM, Abbas R, Burton C, Qazi NA, Nelson MR, et al. Plasma IL-6 as a marker of mycobacterial immune restoration disease in HIV-1 infection. Aids 2003;17:1411-1413.
6. Stone SF, Price P, Keane NM, Murray RJ, French MA. Levels of IL-6 and soluble IL-6 receptor are increased in HIV patients with a history of immune restoration disease after HAART. HIVMed 2002;3:21-27.
7. Li TS, Tubiana R, Katlama C, Calvez V, Ait Mohand H, Autran B. Long-lasting recovery in CD4 T-cell function and viral-load reduction after highly active antiretroviral therapy in advanced HIV-1 disease. Lancet 1998;351:1682-1686.
8. Autran B, Carcelain G, Li TS, Blanc C, Mathez D, Tubiana R, et al. Positive effects of combined antiretroviral therapy on CD4+ T cell homeostasis and function in advanced HIV disease. Science 1997;277:112-116.
9. Narita M, Ashkin D, Hollender ES, Pitchenik AE. Paradoxical worsening of tuberculosis following antiretroviral therapy in patients with AIDS. Am J Respir Crit Care Med 1998;158:157-161.
10. Martinez V, Costagliola D, Bonduelle O, N'go N, Schnuriger A, Theodorou I, et al. Combination of HIV-1-specific CD4 Th1 cell responses and IgG2 antibodies is the best predictor for persistence of long-term nonprogression. J Infect Dis 2005;191:2053-2063.
11. Carson RT, Vignali DA. Simultaneous quantitation of 15 cytokines using a multiplexed flow cytometric assay. J Immunol Methods 1999;227:41-52.
12. Havlir DV, Schrier RD, Torriani FJ, Chervenak K, Hwang JY, Boom WH. Effect of potent antiretroviral therapy on immune responses to Mycobacterium avium in human immunodeficiency virus-infected subjects. J Infect Dis 2000;182:1658-1663.
13. Wendland T, Furrer H, Vernazza PL, Frutig K, Christen A, Matter L, et al. HAART in HIV-infected patients: restoration of antigen-specific CD4 T-cell responses in vitro is correlated with CD4 memory T-cell reconstitution, whereas improvement in delayed type hypersensitivity is related to a decrease in viraemia. Aids 1999;13:1857-1862.
14. Hengel RL, Allende MC, Dewar RL, Metcalf JA, Mican JM, Lane HC. Increasing CD4+ T cells specific for tuberculosis correlate with improved clinical immunity after highly active antiretroviral therapy. AIDS Res Hum Retroviruses 2002;18:969-975.
15. Foudraine NA, Hovenkamp E, Notermans DW, Meenhorst PL, Klein MR, Lange JM, et al. Immunopathology as a result of highly active antiretroviral therapy in HIV-1-infected patients. Aids 1999;13:177-184.
16. Mori T, Sakatani M, Yamagishi F, Takashima T, Kawabe Y, Nagao K, et al. Specific detection of tuberculosis infection: an interferon-gamma-based assay using new antigens. Am J Respir Crit Care Med 2004;170:59-64.
17. Lawn SD, Macallan DC. Hypercalcemia: a manifestation of immune reconstitution complicating tuberculosis in an HIV-infected person. Clin Infect Dis2004;38:154-155.
18. Hirsch CS, Toossi Z, Othieno C, Johnson JL, Schwander SK, Robertson S, et al. Depressed T-cell interferon-gamma responses in pulmonary tuberculosis: analysis of underlying mechanisms and modulation with therapy. J Infect Dis 1999;180:2069-2073.
19. Price P, Morahan G, Huang D, Stone E, Cheong KY, Castley A, et al. Polymorphisms in cytokine genes define subpopulations of HIV-1 patients who experienced immune restoration diseases. Aids 2002;16:2043-2047.

## Claims

1. A method of in vitro diagnosis of Immune Restoration Syndrome (IRS) in patients co-infected with tuberculosis (TB) and HIV, as well as in patients infected with TB alone comprising the step consisting of:
a) incubating peripheral blood mononuclear cells (PBMC) of said patients in a culture medium with mycobacterial antigens including mycobacterial extract referred as PPD (purified protein derivative), and separately with at least one antigen from mycobacterium tuberculosis selected from ESAT-6, CFP-10 and 85B;
b) detecting the level of the Th1 response against said mycobacterial antigens;
wherein an acute increase of Th1 response against said PPD and no or minimal reactivity with ESAT-6, CFP-10 and/or 85B compared to non-stimulated cells or control cells stimulated with non-mycobacterial antigens is indicative of IRS.

2. The method according to claim 1, wherein step b) comprises performing a quantification of antigen-specific T-cells by ELISPOT assay or intra-cellular cytokine staining combined to flow cytometry or fluorescent microscopic examination and quantification.

3. The method according to claim 2, wherein it comprises quantification of mycobacterial-specific Th1 cells producing IFN-γ.

4. The method according to claim 2 or 3, wherein a result above 500 SFC (spot-forming-cells)/10⁶ PBMC, for example a SFC above 500, 1000, or 2000, after subtraction of the mean background obtained with cells alone, is an indication of IRS; while simultaneously no or minimal SFC are detected against ESAT-6, 85B, CFP-10, such as SFC typically below 500/10⁶ PBMC or below 400 or 300.

5. The method according to one of claims 1 to 4, wherein step b) comprises collecting culture supernatants and quantifying at least one Th1 specific cytokine or chemokine, including IL-2, IL-2R, IL-12p40, IL-15, IL-17, IFN-γ, TNF-α, GMCSF, MIP-1α, MIP-1β, MCP-1, MIG, RANTES, IP-10.

6. The method according to claim 5 which is performed using a multiplexed sandwich immunoassay or an individual cytokine-specific sandwich immuno-assay.

7. The method according to claim 5, wherein IFN-γ production is quantified by ELISA.

8. The method according to one of claims 1 to 7, wherein peripheral blood mononuclear cells in step a) are incubated from about 6 to 72 hours.

9. The method according to one of claims 1 to 8, wherein peripheral blood mononuclear cells in step a) are incubated with PPD at about 1µg/ml and separately with ESAT-6 recombinant protein at about 1 µg/ml.

10. The method according to one of claims 1 to 9, wherein peripheral blood mononuclear cells in step a) are incubated with PPD at about 1µg/ml and separately with CFP-10 and/or 85B.

11. The method according to one of claims 1 to 10, wherein control cells are selected from cells incubated with CMV extracts, HIV-1 p24, phytohaemagglutinin and medium alone.

12. The method according to one of claims 1 to 11, which is performed in the course of Highly Active Antiretroviral Therapy (HAART) in HIV positive patients co-infected with tuberculosis.

13. The method according to one of claims 1 to 12, which is performed in patients receiving simultaneous or sequential HAART and tuberculosis treatment.

14. The method according to one of claims 1 to 11, which is performed in patients with TB alone.

15. A diagnostic kit of Immune Restoration Syndrome (IRS) in patients co-infected with tuberculosis (TB) and HIV as well as in patients with TB alone, comprising mycobacterial antigens including mycobacterial extract referred as PPD (purified protein derivative), and at least one antigen from mycobacterium tuberculosis selected from ESAT-6, CFP-10 and 85B.

16. The diagnostic kit according to one of claims 15 further comprising at least one anti-cytokine/chemokine antibody specific to the Th1 immune response bound to a solid support, such as anti-IFN-γ antibodies.

17. The diagnostic kit according to claim 16 wherein the solid support is an ELISPOT plate.

18. The diagnostic kit according to claim 16 wherein the solid support is an ELISA plate.

19. The use of the kit according to one of claims 15 to 18 for detecting Immune Restoration Syndrome (IRS) in patients co-infected with tuberculosis (TB) and HIV in course of HAART and tuberculosis treatment.

20. The use of the kit according to one of claims 15 to 18 for detecting Immune Restoration Syndrome (IRS) in patients with TB alone.
